# EUROPEAN PATENT SPECIFICATION

(11) **EP 0 210 029 B1**
(45) Date of publication and mention of the grant of the patent: **01.06.1994**
(21) Application number: 86305373.2
(22) Date of filing: 14.07.1986
(51) Int. Cl.: C07K 15/00, C12P 21/00, C12N 15/00, C12N 5/00, G01N 33/573, G01N 33/577

(54) **Monoclonal antibodies to human plasma prekallikrein and methods of preparing and using same**
Monoklonale Antikörper gegen menschliches Plasma-Prekallikrein und Verfahren zu ihrer Herstellung und Verwendung
Anticorps monoclonaux contre la prékallicréine de plasma humain et méthodes pour leur préparation et leur utilisation

(30) Priority: 12.07.1985 US 754800; 08.07.1986 US 883218
(43) Date of publication of application: 28.01.1987
(73) Proprietor: Temple University of the Commonwealth System of Higher Education, Philadelphia PA 19122 (US)
(72) Inventor: Veloso, Dulce C., Philadelphia, PA 19144 (US); Colman, Robert W., Moylan, PA 19065 (US)
(74) Representative: Ackroyd, Robert

(56) References cited:
- EP-A- 0 080 279
- EP-A- 0 085 402
- EP-A- 0 100 395
- CHEMICAL ABSTRACTS, vol. 102, no. 1, 07 January 1985, Columbus, OH (US); G.S. BEDI et al., p. 409, no. 4284t#
- PATENT ABSTRACTS OF JAPAN, vol. 7, no. 274 (P-241)[1419], 07 December 1983#
- PATENT ABSTRACTS OF JAPAN, vol. 10, no. 305 (P-507)[2361], 17 October 1986#
- PROCEEDINGS OF THE NATL. ACADEMY OF SCIENCE USA, vol. 82, August1985; A. DE AGOSTINI et al., pp. 5190-5193#

## Description

### Field Of The Invention

This invention relates to hybrid cell lines for production of monoclonal antibodies to human plasma prekallikrein, a protein which is found in nearly all human blood. The invention also relates to the antibody produced by these cell lines, and to diagnostic, therapeutic and biochemical methods and compositions using the same.

### Background Of The Invention

Prekallikrein is a single-chain glycoprotein which is present in human blood in at least two forms having molecular weights of 85 kDa and 88 kDa, respectively as reported in Hematology, p. 1210, McGraw-Hill, Inc., 1983, USA. Prekallikrein is one of four plasma proteins which are junction-initiating and/or modulatory proteins for coagulation, fibronolysis, complement activation, prorenin activation, and possibly other biochemical pathways occurring in the plasma. Colman, R. W., J. Clin. Inv. 73: 1249-1253 (1984). The other junction-initiating or modulatory plasma proteins are (1) factor XII, also known as Hageman factor, (2) high molecular weight kininogen and (3) Cl-inhibitor.

Prekallikrein and factor XII are plasma serine protease zymogens. Conversion of prekallikrein to the active enzyme kallikrein is initiated by autoactivation of factor XII to factor XIIa in the presence of negatively charged surfaces. Factor XIIa (or factor XIIf) cleaves prekallikrein into kallikrein. Kallikrein activates factor XII to produce additional factor XIIa. Kallikrein consists of two polypeptides linked together by one or more disulfide bridges. One of the kallikrein polypeptides known as the "heavy chain" has a molecular weight of 60 kDa. One other polypeptide known as the "light chain" has two variants, 33 kDa and 37 kDa, respectively, depending on the form of prekallikrein from which they originate. The light chain contains the enzyme active site. The 33 kDa kallikrein light chain originates from 85 kDa prekallikrein, while the 37 kDa variant is from the 88 kDa form of prekallikrein.

Prekallikrein is present in blood as a non-covalent complex with high molecular weight kininogen. The latter is a cofactor for both factor XIIa and kallikrein. It is also a substrate of kallikrein. Kallikrein cleaves high molecular weight kininogen into four fragments: three polypeptides and the potent vasodepressor peptide bradykinin. Other substrates of kallikrein include plasminogen and prorenin. In addition, kallikrein stimulates neutrophil aggregation and degranulation.

Cl-inhibitor and alpha₂-macroglobulin are the two major plasma protease inhibitors. Cl-inhibitor inhibits kallikrein by forming a covalent complex with it. Alpha₂-macroglobulin inhibits kallikrein by combining with kallikrein to form a noncovalent complex. Cl-inhibitor and alpha₂-macroglobulin thus regulate the activity of kallikrein in the body. A deficiency of Cl-inhibitor gives rise to the classic disease hereditary angioedema and a decrease in the level of functional prekallikrein without change in prekallikrein-kallikrein antigen. Activation of prekallikrein may be inferred from a drop in its plasma level, by formation of kallikrein-Cl-inhibitor complex, or by the release of bradykinin into the circulation, concomittant with cleavage of plasma high molecular weight kininogen. The appearance of these signals have been demonstrated in a variety of diseases, most notably gram negative septicemia. Colman, R.W., J. Clin. Inv. 73: 1249-1253 (1984). This affliction is a major cause of death and disability among hospitalized patients, with an incidence of several hundred thousand cases each year. Hypotension and hemorrhage are frequent complications due to activation of the contact phase of blood coagulation. Endotoxin infusion leads to activation of factor XII, which in turn cleaves prekallikrein to the active form kallikrein, resulting in a drop in the level of prekallikrein in the plasma. When kallikrein inhibitors are consumed, the concentration of the active form, kallikrein (as distinguished from the inactive form, prekallikrein), is increased. Early detection of this change in plasma prekallikrein and kallikrein levels could allow intervention with treatment before the occurrence of irreversable shock.

Kohler and Milstein, Nature 256, 493-497 (1975) were the first to describe the fusion of myeloma cells to immune spleen cells from mice to generate continuous cell lines. These hybrid cell lines, or hybridomas, have characteristics that neither the parental myeloma cells not parental immune spleen cells possess. Hybridomas are capable of continuously producing homogeneous (monoclonal) antibodies which recognize a single antigenic determinant. Prior to the work of Kohler and Milstein, only polyclonal antisera could be obtained, which are not capable of continuously producing identical antibodies. Polyclonal antibodies recognize a number of different antigenic determinants. Because of homology among the polypeptides containing the active site of covoral serine proteases, and homology between the heavy chains of prekallikrein and factor XI, thorough characterization of antibodies is necessary for diagnostic utility, which could be difficult and time-consuming in the case of polyclonal antibodies.

Although techniques for the production of hybridomas are now extensively described in literature, e.g., Monoclonal Antibodies, Hybridomas: A New Dimension In Biological Analysis, R. H. Kennett, T. J. McKearn, and K. B. Bechtol, eds., Plenum Press, New York and London (1980), there is no general method for obtaining successful monoclonal antibody-producing hybridomas which can be used with all antigens. Fusion techniques must be varied in each case to obtain hybridomas producing monoclonal antibody to the desired antigen. In order to obtain antibodies specific to a single antigen, laborious purification techniques are required to provide highly purified antigen for either immunization or screening. The production of monoclonal antibodies for any given antigen is still a highly empirical process.

There have been no previous reports of monoclonal antibodies against human plasma prekallikrein, although plasma prekallikrein is present in all humans with very few exceptions. Prekallikrein and kallikrein are detected in patient blood by means of coagulant and immunochemical assays using polyclonal antisera. The dearth of literature accounts of monoclonal antibodies to prekallikrein is no doubt due to difficulties in the purification of antigen and/or the lack of success in preparation of suitable hybridomas.

### Summary Of The Invention

According to the present invention, novel hybridomas have been prepared providing cell lines producing monoclonal antibodies which specifically bind to an antigenic determinant of human plasma prekallikrein. Each hybridoma comprises a cell hybrid formed by fusion of cells from a myeloma line and spleen cells from a donor previously immunized with human plasma prekallikrein. The hybridomas are, respectively, ATCC #HB-8862 through #HB-8865. Each antibody so produced is specific for an antigenic determinant of human plasma prekallikrein. For at least #HB-8862, -8863 and -8864, the antigenic determinant of prekallikrein so recognized is also shared by plasma kallikrein and kallikrein-Cl-inhibitor complex. The antigenic determinant is contained on the kallikrein heavy chain. The purified monoclonal antibody contains essentially no other anti-human immunoglobulin. The hybridomas may be cultured in vitro to produce antibodies.

The hybrid cell lines of the present invention may be prepared by first immunizing mice with purified human plasma prekallikrein. The spleen cells are then removed and a suspension thereof is made. The spleen cells are fused with mouse myeloma cells in the presence of a fusion promotor. The fused cells are diluted and cultured in separate wells in a medium which will not support the unfused myeloma cells. The supernatant in each well is assayed for the presence of antibody to human plasma prekallikrein by enzyme-linked immunosorbent assay ("ELISA"). Hybridomas secreting antibody which binds to human plasma prekallikrein are selected and cloned.

The hybridomas are cultured in a suitable medium and the antibody is recovered from the supernatant. Alternatively, the clones are transferred intraperitoneally into mice, and the resulting malignant ascites and serum containing the desired antibody are harvested.

Methods for detecting the level of human plasma prekallikrein in specimens of interest are provided. The specimen is incubated with excess Cl-inhibitor to convert all kallikrein to kallikrein-Cl-inhibitor complex. The complex is absorbed from the specimen, preferably by affinity chromatography using a commercially available human-anti-Cl-inhibitor antibody. The specimen is then contacted with monoclonal antibody according to the present invention, which binds to an antigenic determinant of prekallikrein. The material bound by the antibody is measured by standard assay means.

According to another method, levels of all three proteins, that is, prekallikrein, kallikrein, and kallikrein-Cl-inhibitor complex, are measured. A specimen, preferably blood plasma, is divided into three aliquots, A, B, and C. Kallikrein-Cl-inhibitor complex is adsorbed from A, preferably by affinity chromatography using a commercially available anti-human-Cl-inhibitor antibody. Alliquot B is incubated with excess Cl-inhibitor to convert all kallikrein in B to kallikrein-Cl-inhibitor complex. Kallikrein-Cl-inhibitor complex is then adsorbed from B, preferably by affinity chromatography using a commecially available anti-human-Cl-inhibitor antibody. Each of A, B and C is then separately contacted with a monoclonal antibody according to the present invention which binds a single antigenic determinant shared by prekallikrein, kallikrein and kallikrein-Cl-inhibitor complex. The material bound by said antibody in each of A, B and C may then be measured by an assay means.

The material bound in B comprises prekallikrein. The material bound in A comprises prekallikrein plus kallikrein. The amount of kallikrein in the specimen is thus derived by subtracting the amount of material bound in B (prekallikrein) from the amount of material bound in A (prekallikrein plus kallikrein). The material bound in C comprises prekallikrein, kallikrein, and kallikrein-Cl-inhibitor complex. Subtraction of the amount of material bound in A, from that bound in C, gives the amount of kallikrein-Cl-inhibitor complex in the specimen.

In another embodiment, samples of a specimen are divided into two aliquots A and B. B is incubated with excess Cl-inhibitor to convert all kallikrein to kallikrein-Cl-inhibitor complex. The proteins of each of A and B are then separated according to molecular weight. The thus-separated proteins are contacted with a monoclonal antibody of the present invention and the amount of protein bound by the antibody is measured by an assay means. Measuring the amount of about 185 kDa protein from A bound by the antibody indicates the amount of kallikrein-Cl inhibitor complex in the specimen. Measuring the amount of about 85-88 kDa protein from B bound by the antibody indicates the amount of prekallikrein in the specimen. Measuring the amount of about 85-88 kDa protein from A bound by the antibody indicates the total amount of prekallikrein plus kallikrein in the specimen. Subtraction of the former amount from the latter amount provides the amount of kallikrein in the specimen.

The antibodies may be bound to an immobilized matrix and used to purify prekallikrein, kallikrein or kallikrein-Cl-inhibitor complex, and to generate prekallikrein-deficient plasma. Matrices of this type charged with prekallikrein may be used to purify high molecular weight kininogen, which binds prekallikrein. Undesirable amounts of prekallikrein or kallikrein may be therapeutically removed from the blood in this manner.

It is, accordingly, an object of this invention to provide hybridomas which produce antibodies against human plasma prekallikrein.

Another object of this invention is to provide essentially homogeneous antibodies against this antigen.

It is another object of this invention to provide a method for measuring the level of human plasma prekallikrein in specimens of interest, such as human plasma.

It is an object of this invention to provide a method for measuring the level of human plasma prekallikrein, kallikrein, and kallikrein-Cl-inhibitor complex.

It is an object of this invention to provide a method for purifying human plasma prekallikrein.

It is another object of this invention to provide a method for separating the light and heavy chains of kallikrein.

It is an object of the invention to provide an in vivo method for reducing the level of functional kallikrein.

It is an object of the present invention to provide a method of therapy for removing excessive amounts of plasma prekallikrein and kallikrein from the blood.

It is a further object of this invention to provide a method for purifying high molecular weight kininogen.

It is an object of this invention to provide a method for purifying kallikrein-Cl-inhibitor complex.

Other objects and advantages of this invention will become apparent from the present disclosure.

Each of the four subject hybridomas are identified herein by the same number assigned to the antibody produced thereby. Thus, for example, the designation "6A6" pertains to both the hybridoma 6A6-B3-E12 and the monoclonal antibody produced by this hybridoma. The particular material referred to, that is, hybridoma versus antibody, is apparent from the context.

The subject hybridomas were deposited in the American Type Culture Collection, 12301 Parklawn Drive, Rockville, Maryland 20852, and were given the following ATCC accession numbers: #HB-8863 for 6A6-B3-E12; #HB-8864 for 13Hll-Fll-B7; #HB-8862 for 13Gll-B10-D6; and #HB-8865 for 10B6-F6-D5. #HB-8862 and 8863 were deposited on July 9, 1985; #HB-8864 and 8865 were deposited on July 10, 1985.

### Brief Description of the Figures

Figure 1 is a plot of the inhibition of the amidolytic activity of kallikrein (or prekallikrein following activation by factor XIIf) by the antibodies of the present invention as determined by the hydrolysis of the synthetic substrate D-proline-phenylalanine-arginine-p-nitroanilide (D-Pro-Phe-Arg-pNA).

Figure 2 is a plot of the inhibition of kaolin activation of human plasma by the antibodies of the present invention as determined by the hydrolysis of the synthetic substrates D-Pro-Phe-Arg-pNA or benzoyl-proline-phenylalanine-arginine-p-nitroanilide (Bz-Pro-Phe-Arg-pNA).

Figure 3 is a plot of the inhibition of the procoagulant activity of prekallikrein in plasma with the antibody 13Gll. Similar patterns were observed with the 6A6 and 13Hll antibodies.

### Detailed Description Of The Invention

The monoclonal antibodies of the present invention react with human plasma prekallikrein. Monoclonal antibodies produced by one of the four cell lines, 10B6, has not been further characterized. The remaining cell lines, 6A6, 13Hll and 13Gll, were found to recognize not only human plasma prekallikrein, but also kallikrein, specifically the heavy chain of kallikrein. They also recognize kallikrein-Cl-inhibitor complex. They produce an IgG monoclonal antibody of subclass IgG₁, kappa light chain. The antibodies have a molecular weight of 160 kDa, which upon reduction yield 50 kDa and 28 kDa fragments. They recognize nonreduced human plasma prekallikrein and kallikrein, but reduction with mercaptoethanol decreases the strength of the reaction. Since fewer of the epitopes appear to be available after reduction, binding of antibodies 6A6, 13Hll and 13Gll to the kallikrein heavy chain was observed with large amounts of antigen.

Prekallikrein is present as a collection of microheterogeneous monomer glycoproteins. Each monomer has a molecular weight of 85 kDa or 88 kDa as determined by migration on SDS polyacrylamide gels. The various collections of microheterogeneous monomers have isoelectric points of 7.9, 8.3, 8.6, 8.8, 9.1, 9.3 and 9.5. Hojima et al, J. Biol. Chem. 260, 400 (1985). The species with the isoelectric points 8.6, 8.8, 9.1 and 9.3 are the most abundant.

Human plasma prekallikrein was purified according to a method relying on the difference between isoelectric points, and therefore differences in dissociation constants between prekallikrein and other plasma proteins. The procedure does not eliminate all irrelevant immunoglobulins, but these contaminants can be removed by affinity chromatography, such as immobilized anti-human IgG chromatography, by protein A chromatography, or by binding with high molecular weight kininogen. Other purification methods may be used including binding with immobilized monoclonal antibodies according to the present invention.

Mice are immunized with purified human plasma prekallikrein. BALB/c AnSkh mice are preferred, although other strains may be used. The immunization schedule and concentration of antigen administered should be such so as to produce useful quantities of suitably primed splenocytes.

Upon completion of the immunization regimen more completely described below, the mice are sacrificed and their spleens removed. A suspension of splenocytes in a suitable medium is prepared. Approximately 2.5 - 5 ml of medium per spleen is sufficient. The protocols for in vitro cell suspension are well established.

The spleen cells are fused with mouse myeloma cells by means of a fusion promotor. The preferred fusion promotor is polyethylene glycol, molecular weight 1300 - 1600. Other promotors may be used. The mouse myeloma cell line is preferably one of the "drug-resistant" types, to enable selection of hybrids. The most frequently used class of myelomas are the 8-azaguanine-resistant cell lines, which are widely known and available. These cell lines lack the enzyme hypoxanthine guanine phosphoribosyl transferase and therefore do not survive in "HAT" (hypoxanthine aminopterin-thymidine) medium. The use of myeloma cells with different genetic deficiencies (e.g., other enzyme deficiencies, drug sensitivities, etc.) that can be selected against in media supporting the growth of genotypically competent hybrids is also possible. Additionally, it is preferred that the myeloma cell line does not itself produce any antibody, although in some circumstances, secreting myeloma cell lines may be preferred.

While the preferred fusion promoter is polyethylene glycol of average molecular weight 1300-1600 (available from ATCC), other known fusion promotors may be used.

Fusion of cells may be carried out in an adherent monolayer, such as according to the method described by T. J. McKearn in "Fusion Of Cells In An Adherent Monolayer" Monoclonal Antibodies: Hybridomas: A New Dimension In Biological Analysis, (Kennett, R.H., McKearn, T.J., and Bechtol, K.B., eds.), Plenum Press, New York and London, 368-369 (1980). Other fusion techniques may be employed. A cell ratio of 2-3:1 spleen cells per myeloma cell may be used. This ratio may be varied depending on the source of spleen or myeloma cells.

A mixture of unfused myeloma cells, unfused spleen cells and fused cells are distributed for culturing in separate compartments (e.g., the wells of a 96-well microliter plate) in a selective medium in which the unfused myeloma cells will not survive. Distribution of the cells may be by resuspension in a volume of diluent which is statistically calculated to isolate a desired number of cells per compartment. See, McKearn, T.J., "Cloning of Hybridoma Cell Lines by Limiting Dilution in Fluid Phase" in Monoclonal Antibodies, p. 374.

When HAT is used as the medium, unfused 8-azaguanine-resistant myeloma cells will not grow. Unfused spleen cells will normally die after a few days, since they are non-malignant. Culturing proceeds for a time sufficient to allow their death. Fused cells continue to reproduce and grow in the selective medium.

The supernatant in each container or compartment having hybrid cell growth is screened and evaluated for the presence of antibody to human plasma prekallikrein. Any suitable antibody-binding detection method may be used, e.g., enzyme-linked immunosorbent assay. Other assays, such as radioimmunoassay, may be advantageously employed.

After selection and cloning, monoclonal antibody to human plasma prekallikrein may be produced by in vitro culturing of the hybridomas or by in vivo peritoneal exudate induction in mice. The first method will yield monoclonal antibody of higher purity. The antibody is recovered from the supernatant essentially free of undesired immunoglobulin. Antibody concentrations of 25-50 micrograms/ml are possible by this method. In growth media containing serum (such as fetal calf serum) a small amount of other immunoglobulin is present.

Where concentrations of antibody larger than those obtained by in vitro culturing of hybridomas are required, the subject hybridomas may be injected into the peritoneal cavity of syngeneic or semi-syngeneic mice. After a suitable period of incubation, the hybridoma causes the formation of antibody-secreting tumors, which will produce 4-10 mg of antibody per ml of blood or peritoneal exudate of the injected mouse. Since mice have normal antibodies in their blood and ascites, a contamination of about 5% from the host mouse is inevitable. Purification of ascites monoclonal antibody may remove these contaminants. The resultant antibody is of high titer, being active at dilutions of 1:300,000 or higher.

The following is one typical procedure for preparing hybrid cell lines according to the present invention, which is not intended to be limited to the same.

### Preparation of the Immunogen

Human plasma prekallikrein was purified according the procedure of Scott et al., Eur.J.Biochem. 100, 77 (1979), with modifications. All operations were carried out in plastic material since glass activates the coagulation system. A total of four units of fresh blood from four healthy human individuals was collected in fractions of 90 ml into tubes containing 10 ml of an anticoagulant solution of 3.8g of sodium citrate and 2.5g of dextrose per 100 ml also containing the following proteinase inhibitors: ethylenediamine tetraacetate (0.37g), benzamidine (0.16g), aprotinin (3 TIU, Sigma), hexadimethrine bromide (10mg) and soybean trypsin inhibitor (40mg). The blood treated in this manner was spun at 2,000 rpm for 10 minutes to separate red cells, and then centrifugated at 10,000 rpm for 20 minutes to separate platelets. The plasma obtained (800 ml) was treated with 0.2 mM (final concentration) of diisopropylfluorophosphate and 3 TIU of aprotinin. The plasma, after addition of 80 mg of hexadimethrine bromide, was stirred for 30 minutes at room temperature with 800 ml of a slurry of QAE-Sephadex® A-50 (Pharmacia) made by swelling the resin in a buffer containing 0.01 M Tris, 0.001 M benzamidine, 0.001 M ethylenediaminetetraacetate and 0.02% sodium azide adjusted to pH 8 with HCl (buffer A). After stirring, the mixture of plasma-resin was filtered through Whatman no. 1 filter paper. The filtrate was adsorbed to another 800 ml of a QAE-Sephadex® slurry made with buffer A and then treated and filtered as above. QAE-Sephadex treatment was repeated again but this time without the hexadimethrine and with 1.5 M urea (final concentration). The final filtrate was diluted with water to the conductivity of buffer A and then stirred for 75 minutes at 4°C with 700 ml of a slurry of SP-Sephadex® C-50 (Pharmacia) made by swelling the resin with buffer A. The slurry was loaded onto a chromatography column.

Sephadex® chromatography, and the operations which followed, were at 4°C, except that protein A/anti-(human IgG) chromatography was at room temperature.

Elution from the Sephadex column was with a linear NaCl gradient from 0 to 0.17 M NaCl (800 ml/chamber). Prekallikrein (detected by its coagulant or amidolytic activity as reported by Scott et al., Eur. J. Biochem. 100, 77 (1979)), eluted in partially separated form from most of the plasma proteins, but still contained IgG, factor XI and beta₂-glycoprotein I. For separation of these latter two proteins, and for further purification of prekallikrein from IgG, the pooled prekallikrein-containing fractions of the previous chromatography were concentrated and adjusted to the conductivity of buffer B. Buffer B consisted of 0.1 M sodium acetate, 0.1 M NaCl, 0.001 M ethylenediaminetetraacetate, 0.001 mM benzamidine and 0.02% sodium azide, adjusted to pH 5.3 with acetic acid. The eluate of the first SP-Sephadex® chromatography after the above adjustment was applied to a second SP-Sephadex® column (about 300 ml bed volume) equilibrated with buffer B. Elution was with a linear gradient 0 to 0.35 M NaCl in buffer B (400 ml/chamber). The fractions containing prekallikrein (factor XI should be absent) were pooled, concentrated and stored at -70°C.

For removal of the remaining IgG contaminants, aliquots (1 to 2 ml) of the concentrated fractions were adjusted to pH 8 with 1 M Tris and then applied to a protein A-Sepharose column (3 ml bed volume) placed in tandem with an anti-(human-IgG)-agarose column (6 ml bed volume). Elution of pure prekallikrein was with 10 mM Tris-HCl/0.15 M NaCl, pH 7.5, as the buffer.

### Immunization

Four male or female BALB/c AnSkh mice, 8-10 weeks old (Temple University, Skin and Cancer Hospital,) were immunized subcutaneously with 35 micrograms of protein/mouse in complete Freund's adjuvant (week 0) and then again subcutaneously with 35 micrograms of protein/mouse in incomplete Freund's adjuvant at week 5. Blood was removed and screened at week 7 for antibodies to the immunogen using enzyme-linked immunosorbent assay ("ELISA"). At week 11, 50 micrograms of immunogen/mouse in 0.15 M sodium chloride were intraperitoneally injected. Four days later, blood was removed from the retro-orbital plexus of each mouse under light anesthesia, and the two strongest positive mice were selected as spleen donors. The spleens of these animals were asceptically removed and placed in tissue cultured dishes (15 x 60 mm) containing Hank's balanced salt solution ("HBSS", Gibco, Grand Island, NY) to which 50 micrograms/ml of gentamycin or "PEN/STREP" (Gibco) were added. The latter is a mixture of pennicilin and streptomycin. The spleens were then transferred into other culture dishes containing HBSS. The spleens were teased apart with sterile forceps and then transferred into a centrifuge tube which was placed in ice for two minutes to allow large debris to settle. The cell-suspension was transferred into another centrifuge tube and spun for ten minutes at 1200 rpm. After discarding the supernatant, the cells were resuspended in 5-10 ml of 0.17 M NH₄Cl (ice cold) and placed in ice for five minutes with occasional mixing in order to lyse red blood cells. The cell suspension was gently underlaid into 10 ml of 1:1 dilution of HBSS:normal serum and centrifuged at 1200 rpm for ten minutes. Fetal calf serum ("FCS") may be used as the normal serum. The cells were then washed thrice in Dulbeco's Modified Eagle's Medium ("DME", Gibco). The number and viability of cells was then determined.

SP2/O-Ag14 myeloma cells used in the hybridization procedure were washed in the same way as the unlysed splenocytes.

### Preparation of Splenocyte Feeder Layers

On the day of fusion, non-immune splenocytes from the same mouse strain as immunized above were processed according to the same procedure without immunization and without washing in DME. These non-immune splenocytes were used to prepare feeder layers as follows. The non-immune cells were resuspended in DME + HAT + 20% FCS to a density of 2-4 x 10⁶ cells/ml. These cells were seeded onto 96-well plates (1-2 x 10⁵ cells/well) and incubated in 5% CO₂ at 37°C overnight as a sterility check before plating out hybrid cells

### Hybridization

Fusion was carried out as follows. 1.5 ml of immune splenocytes and 1.5 ml of SP2/O-Ag14 cells were pipeted onto a concanavalin A-coated plate. The cell concentration of each cell type was adjusted so that the ratio of splenocytes to SP2/O-Ag14 cells was 2-3:1, with a total of 7-10 x 10⁷ cells/plate. The plates were then incubated in 5% CO₂ at 37°C for 45-60 minutes to allow for attachment of the cells to concanavalin A. Fusion was performed by adding 1 ml of a 50% DME:PEG solution to each plate, drop by drop. The plates were left standing for 15 seconds after the addition of the first drop. The cells were then washed twice with 5 ml of DME. Following addition of 5 ml of DME + 20% FCS/plate, the cells were incubated overnight.

### Selection and Growth of Hybridomas

Following overnight incubation, the cells from the above hybridization procedure were transferred into centrifuge tubes and spun at 1500 rpm for 15 minutes. The supernatants were discarded. The cells from each tube were suspended in 40-45 ml of DME + HAT + 20% FCS and transferred into the 96-well plates (0.1 ml cell suspension/well) containing non-immune splenocyte feeder layers as prepared in "Preparation of Splenocyte Feeder Layers", see above. The plates were cultured with 10% CO₂ at 37°C in a humid atomosphere. The cells were allowed to grow for 3-5 days, after which an additional 0.1 ml of DME + HAT + 20% FCS were added to each well. Hybrids were checked daily. Three to four weeks after fusion, the cells were switched to DME + HAT + 10% FCS (no aminopterin). Hybridoma cultures with antibody reactive to the immunogen were selected and cloned by a limiting dilution technique. McKearn, T.J. "Cloning of Hybridoma Cells By Limiting Dilution And Fluid Phase" in Monoclonal Antibodies, p. 374. Cells from the four strongest positive cultures were injected intraperitoneally (about 2x10⁶ cells in 0.5 ml PBS/mouse) into four BALB/c mice which had been primed 10-14 days previously with 0.5 ml of pristane (2,6,10,14-tetramethylpentadecane). The cultures were designated 6A6, 13Hll, 13Gll, and 10B6. After 7-14 days, the blood was removed from the retro-orbital plexus of each mouse under light ether anesthesia and the tumor-induced ascites fluid was harvested. The antibody titer of 6A6, 13Hll and 13Gll was determined in the ascites fluid using pure immunogen and ELISA.

### Purification of Antibody

The ascites fluids were purified and assayed as follows. Fluids were desalted with a desalting gel filtration column (Sephadex G-25) using a buffer of 20 mM Tris-HCl pH 7.8. The protein-containing fraction eluted from this column was applied to a "MONO-Q" ion exchange column (Pharmacia) which was adapted to a fast protein liquid chromatography machine (Pharmacia). The antibody was eluted by a step gradient generated by buffer A (20 mM Tris-HCl) and buffer B (20 mm Tris-HCl+1.0 M NaCl). Programming was planned to give an isocratic elution of the desired monoclonal antibody. The antibody was detected with mouse anti-IgG by Outcherlony immunodiffusion. The positive fractions were rechromatographed by the same technique, except that the buffers were pH 7.5. Rechromatography yielded a microheterogeneous peak of the desired antibody. Further rechromatography under chromatofocusing conditions, pH 7-4, gave only one microheterogeneous peak for 6A6, 13Hll, or 13Gll, with elution pH's for each antibody as shown in Table 1. The sub-class of the monoclonal antibodies was determined with a mouse immunoglobulin subtype identification kit (Mannheim Boehringer).

**TABLE 1**

| Clone | ATCC No. | Clone Titer | Antibody Sub-class | Chromatofocusing pH elution |
|---|---|---|---|---|
| 6A6 | HB-8863 | >300,000 | IgG₁,k | 5.66 |
| 13Hll | HB-8864 | 300,000 | IgG₁,k | 5.56 |
| 13Gll | HB-8862 | >300,000 | IgG₁,k | 5.76 |
| 10B6 | HB-8865 | -- | -- | -- |

The specificity of the monoclonal antibody 13Gll for prekallikrein and for kallikrein (with or without separation of the two polypeptide chains by mercaptoethanol) was demonstrated either by applying the proteins directly to the nitrocellulose membrane, or after separation of the proteins by 10% SDS-PAGE according to Laemmli, Nature 227, 680 (1970) and electrophoretic transfer to nitrocellulose membranes according to Towbin, et al., Proc. Natl. Acad. Sci. U.S.A. 76, 4350 (1979). Binding of antigen by antibody 13Gll was detected by ELISA, using peroxidase-conjugated goat-antimouse IgG as the secondary antibody. The results are set forth in Table 2.

**TABLE 2**

| Protein | Direct Application | Electrophoretic Transfer from SDS-PAGE | |
|---|---|---|---|
| | | Non-Reduced | Reduced |
| H. Plasma PK | + | + a | - |
| H. Plasma K | + | + a | |
| H. Plasma K (heavy chain) | | | + a |
| H. Plasma K (light chain) | | | - a |
| B. Pancreas K | | - b | |
| B. Plasma K | | - | |
| H. Plasma factor XI | | - | |
| H. Plasma factor XII | - | - | |
| H. Plasma HMWK | - | - | |
| H = Human B = Bovine PK = Prekallikrein; K = Kallikrein; HMWK = high molecular weight kininogen Reduced or non-reduced = protein treated with or without mercaptoethanol + = positive immunoreaction - = negative immunoreaction a = antibodies 6A6 and 13Hll were also tested, and the reaction was the same as that of 13Gll b = antibody 6A6 was also tested, and reaction was the same as that of 13Gll. | | | |

The four monoclonal antibodies of the present invention all reacted with biochemically pure plasma prekallikrein. The three monoclonal antibodies 6A6, 13Hll and 13Gll were also positive against plasma kallikrein and its heavy chain. Although fewer of the epitopes appeared to be available after reduction of both prekallikrein and kallikiein with mercaptoethanol, binding of antibody 6A6, 13Hll and 13Gll to the kallikrein heavy chain was observed using 10-fold as much kallikrein as compared to binding under non-reducing conditions.

Table 2 indicates the specificity of the antibodies for prekallikrein and kallikrein purified from plasma. The antibodies recognize plasma kallikrein and do not cross-react with kallikrein derived from tissue; nor do they cross-react with the related plasma proteins factor XI, factor XII and high molecular weight kininogen. Specificity for prekallikrein, kallikrein and kallikrein-Cl-inhibitor complex was observed for 13Gll by separation of proteins in normal plasma (and proteins in prekallikrein-deficient plasma to which kallikrein was added) by SPS-PAGE and immunoblotting.

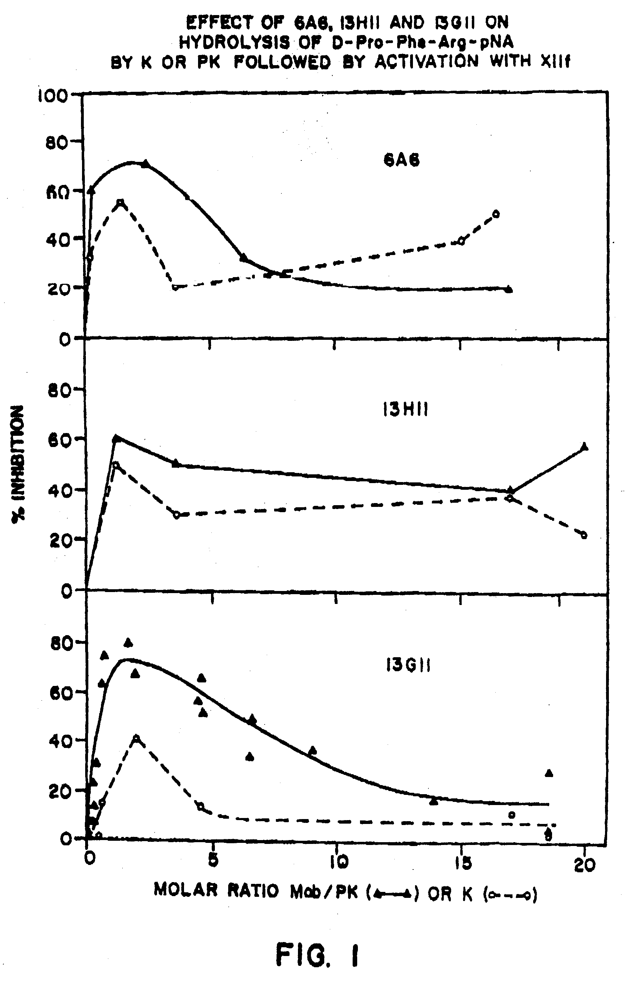

The effect of the instant monoclonal antibodies on the amidolytic activity of prekallikrein and kallikrein was determined from the hydrolysis of the chromogenic substrate D-Pro-Phe-Arg-pNA in pure systems, as follows:
0.3 micrograms of either prekallikrein or kallikrein in 50 microliters of buffer (pH 7.5) containing 1 mg/ml of human serum albumin were incubated at 37°C for 40 minutes with monoclonal antibody in ratios of antibody to prekallikrein (or kallikrein) varying from zero to 15. Where prekallikrein was used, it was activated by factor XIIf after incubation with the monoclonal antibody. Kallikrein was prepared from prekallikrein by activation with factor XII fragment, followed by inactivation of the activated fragment with corn trypsin inhibitor. The amidolytic activity of the plasma protein in the presence or absence of monoclonal antibody was determined spectrophotometrically at 405 nm in 10 microliter aliquots added to 0.3 ml of a 1 mM solution of the chromogenic substrate made in 0.1 M of sodium phosphate/0.15 M NaCl, pH 7.6, prewarmed at 37°C. The results appear in Figure 1. The antibody concentration is as indicated in Figure 1.

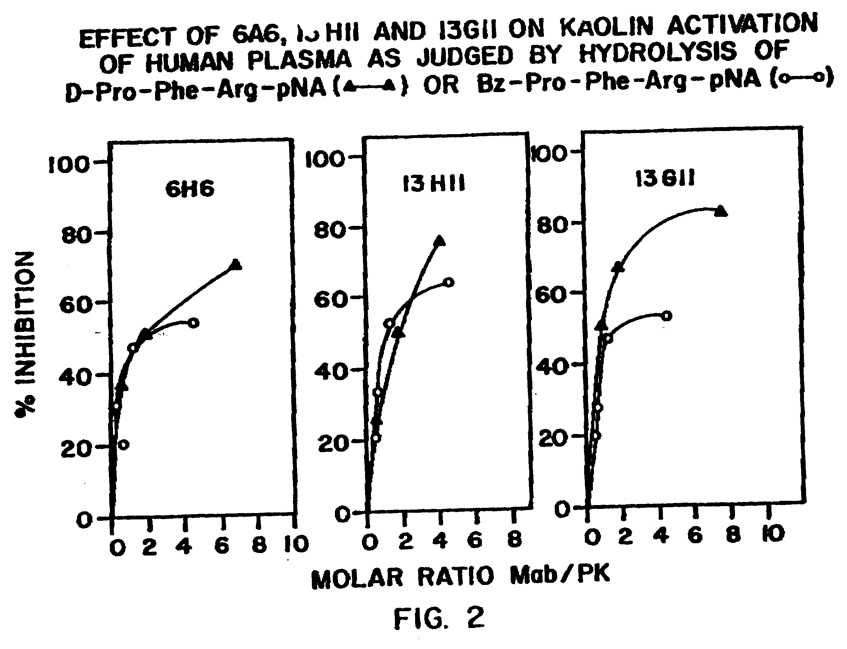

Likewise, the effect of the present monoclonal antibodies on the amidolytic activity of prekallikrein and kallikrein in normal human plasma was determined from the hydrolysis of D-Pro-Phe-Arg-pNA or Bz-Pro-Phe-Arg-pNA. Normal plasma was incubated with or without (control) monoclonal antibody followed by activation with 20 micrograms of kaolin. The antibody concentration is as indicated in Figure 2, assuming 25 micrograms of prekallikrein per ml of normal plasma. In the case of plasma, equilibrium with the antibodies was already reached after 5 min. of incubation.

Figures 1 and 2 demonstrate that low concentration of monoclonal antibody 6A6, 13Hll, and 13Gll affect the function of prekallikrein and kallikrein. Incubation of these monoclonal antibodies with normal plasma in a molar ratio of antibody to prekallikrein up to 5:1 decreased hydrolysis of the two chromogenic substrates tested with a profile slightly different for each antibody (Figure 2). When either kallikrein or prekallikrein was incubated with these antibodies, followed by activation of prekallikrein with factor XIIf, inhibition was maximal at a molar ratio of antibody to antigen of 1 to 5, and decreased with high concentrations of antibody (Figure 1). Inhibition patterns were slightly different for the three antibodies.

The amidolytic activity of kallikrein demonstrated above with chromogenic substrates is a function of the enzyme's protease activity. Protease activity is present in the light chain. Inhibition as indicated above demonstrates that the epitope recognized by the monoclonal antibodies of the present invention, although located on the heavy chain, is sterically close to the protease active center.

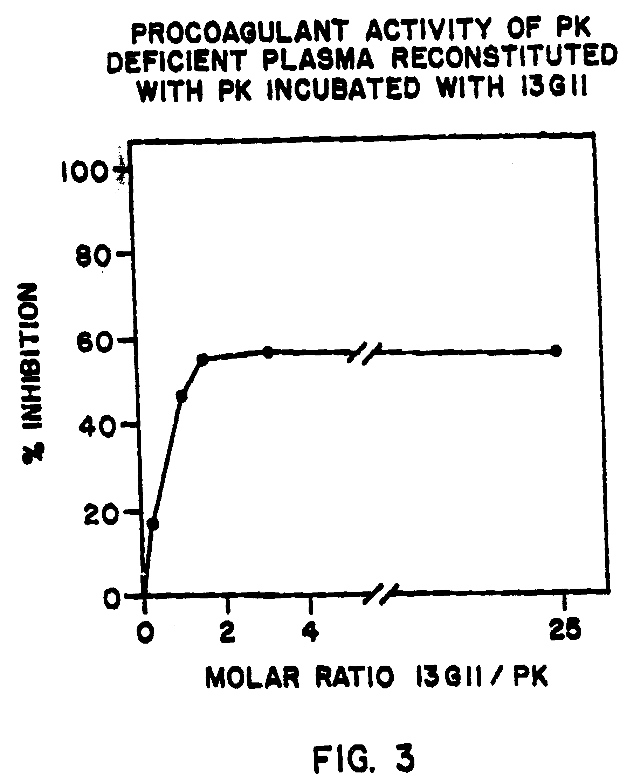

The effect of the monoclonal antibodies of the present invention on the procoagulant activity of prekallikrein was determined. This activity involves both heavy and light chains. Prekallikrein was incubated with each of antibodies 6A6, 13Hll and 13Gll for 30 minutes at 37°C. Thereafter, the prekallikrein was added to prekallikrein-deficient plasma and assayed for clot formation according to the method of Proctor et al., Am. J.Clin.Pathol. 35:212-219 (1961). Figure 3 shows the inhibition curve for antibody 13Gll. It is apparent that procoagulant activity was decreased by about 60% when prekallikrein was incubated with equimolar concentrations of monoclonal antibody before addition to prekallikrein-deficient plasma.

The effect of the instant monoclonal antibodies on kallikrein cleavage of factor XII was established by incubating factor XII, kallikrein and antibody 13Gll at antibody concentrations of 0 to 10 micrograms in a final volume of 40 microliters, pH 7.7. Activation of factor XII was assayed with a chromogenic substrate. The results indicated that antibody 13Gll inhibited activation of factor XII by kallikrein with a pattern similar to that observed for kallikrein in Fig. 1.

The effect of antibody 13Gll on cleavage of prekallikrein by factor XII activated with kaolin in the presence of high molecular weight kininogen, a purified system which mimics the plasma contact activation, was determined by hydrolysis of the substrate D-Pro-Phe-Arg-pNA. The strongest inhibition of hydrolysis of the synthetic substrate was when both prekallikrein and high molecular weight kininogen were present, suggesting a conformational change in the prekallikrein structure, upon binding to high molecular weight kininogen for the formation of a better substrate for the surface-activated factor XII.

The monoclonal antibodies of the present invention are useful in detecting changes in the concentration of prekallikrein, kallikrein and/or kallikrein-Cl-inhibitor complex in human plasma. Thus, the antibodies may be used to diagnose activation of the contact coagulation system, which is signalled by changes in the level of these proteins.

Under normal conditions, essentially only prekallikrein is present in plasma. However, under conditions in which the contact activation system is activated, as for example in the presence of endotoxin, there is a decrease in the level of prekallikrein with a concomitant increase in the levels of kallikrein and/or kallikrein-Cl-inhibitor complex. According to Harpel et al, J. Biol. Chem. 260, 4257 (1985), the Cl-inhibitor present in normal plasma can complex all the kallikrein present in plasma equivalent up to 20% of the normal concentration of prekallikrein. When more than 20% of the normal level of prekallikrein in the plasma is converted to the active enzyme kallikrein, then kallikrein uncomplexed with Cl-inhibitor must be present in the plasma.

The following is one method for measuring the levels of prekallikrein, kallikrein and kallikrein-Cl-inhibitor complex utilizing immunoblotting, followed by enzyme-linked immunosorbent assay.

### Measurement of Prekallikrein, Kallikrein And Kallikrein-Cl-Inhibitor Complex

Plasma is obtained from two ml of blood as described above under "Preparation of the Immunogen", except that no inhibitors are used, and centrifugation is with a Fisher micro-centrifuge, Model 235A for two minutes. Aliquots of 5, 7.5 and 10 microliters of plasma are adjusted to a final volume of 10 microliters each with commercially available prekallikrein-deficient plasma (e.g., George King Biomedicals, Overland Park, Kansas). (Alternatively, prekallikrein-deficient plasma may be prepared by affinity chromatography with immobilized monoclonal antibody of the present invention, which binds prekallikrein). Duplicate samples are prepared in this manner, except that 0.5 micrograms of purified human Cl-inhibitor are added to each duplicate (incubation time 10 min.). The six samples are electrophoresed as described in Lamelli, Nature 227, 680 (1970), with molecular weight standards. The electrophoresed proteins are electrophoretically transferred to nitrocellulose sheets (Schleicher and Schuell), 0.45 micromete pore size, at 8V/cm for 16-20 hr, 4°C according to Burnette, Anal. Biochem. 112, 195 (1981). The nitrocellulose sheets, after brief washing with 10 mM of Tris-HCl buffered to pH 7.5 containing 0.15 M NaCl (TBS), are incubated with TBS containing 5% casein (TBS-C) for two hr at 24°C, followed by incubation with the 13Gll monoclonal antibody (0.8 microgram/ml TBS-C) for two hours. The protein blot is washed with four changes of TBS-C containing 0.05% Nonidet P-40, and then incubated with goat anti-mouse IgG conjugated with horseradish peroxidase (0.040 purpurogallin units/ml) (Sigma). The protein blots are washed as previously with an additional washing with TBS and then placed in a solution of TBS/methanol (100 ml/20 ml) containing 0.015% H₂O₂ and 0.05% 4-chloro-1-naphthol for 15-30 min., to detect the peroxidase-conjugated IgG. Staining is measured by densitometry.

In non-activated normal plasma, only two stained bands are observed corresponding to the 88- and 85-kDa prekallikrein species. The staining intensity as measured by densitometry is proportional to the amount of prekallikrein contained in the sample. The presence of kallikrein-Cl-inhibitor complex is likewise visualized by a stained band at the position corresponding to the molecular weight of the complex, 185 kDa. When high amounts of the active enzyme kallikrein are present, as in activation of the contact system, the intensity of the stained band corresponding to kallikrein-Cl-inhibitor complex will be increased upon addition of exogeneous Cl-inhibitor, with the increase being proportional to the amount of kallikrein present in the sample.

For quantitation, the above procedure is repeated with the same monoclonal antibody, using known amounts of purified prekallikrein and purified kallikrein-Cl-inhibitor complex (purified kallikrein incubated with a molar excess of Cl-inhibitor for 10 min. at 24°C) as standards. These standards, which may span the range from 0.020 to 2.5 micrograms of prekallikrein or kallikrein-Cl-inhibitor complex, are added to a volume of prekallikrein-deficient plasma up to a volume of 10 microliters. A standard curve of staining intensity (as measured by densitometry) versus concentration may then be prepared for each band.

The amounts of kallikrein, prekallikrein and kallikrein-Cl-inhibitor complex in the sample are derived as follows:
For aliquots of sample receiving no exogenous Cl-inhibitor the intensity of the 88- and 85-kDa bands is proportional to the total concentration of prekallikrein plus kallikrein. The intensity of the 185 kDa band is proportional to the concentration of kallikrein-Cl-inhibitor complex. In those aliquots to which exogenous Cl-inhibitor is added, any available kallikrein becomes complexed with Cl-inhibitor. The remaining band intensity at 88 or 85 kDa is thus a measure of the concentration of prekallikrein alone. Kallikrein concentration is then computed as the difference between two measured quantities: [kallikrein] = [kallikrein + prekallikrein] - [prekallikrein].

While measurement of kallikrein, prekallikrein and kallikrein-Cl-inhibitor complex has been exemplified using an immunoblotting procedure followed by enzyme-linked immunosorbent assay, more accurate measurements are possible by further labelling the enzyme-conjugated secondary antibody with an appropriate radiolabel, such as iodine-125. Radio-labelling with iodine-125 is most advantageously conducted to a specific activity of about 10⁷ cpm/nanomol. Appropriate methods for radiolabelling immunoglobulins are known to those skilled in the art, e.g., Helmkamp et al., Int. J. Appl. Radiat. Isotopes 18, 737 (1967). Measurement of specific radioactivity in the bands developed by the above staining procedure provides a more accurate determination of protein concentration than densitometry alone.

The procedure is most advantageously performed using minielectrophoresis and miniblotting apparatus, with incubation of the electrophoresed gels with TBS-C and monoclonal antibody-containing solution, each for 45 minutes at 37°C. Quantitation of prekallikrein, kallikrein and/or kallikrein-Cl-inhibitor complex can thus be efficiently achieved between 3 to 4 hours.

While the above method has been exemplified using electrophoresis to separate the component proteins of the specimen of interest prior to immunoreaction with monoclonal antibody, it is contemplated that other appropriate means for separating proteins according to molecular weight may be advantageously susbstituted, e.g., gel filtration, thin layer chromatography, differential centrifugation, sucrose gradient density centrifugation, etc.

Quantitation of prekallikrein, kallikrein and/or kallikrein-Cl-inhibitor complex in specimens may also be advantageously conducted without separating the component proteins by electrophoresis. In general, a monoclonal antibody of the present invention, which recognizes an antigenic determinant shared by prekallikrein, kallikrein and kallikrein-Cl-inhibitor complex, is contacted with specimen divided into three aliquots A, B and C. Aliquot A is created by passing an aliquot of specimen through a column containing any of the commercially available anti-human-Cl-inhibitor antibodies (e.g., Calbiochem, San Diego, CA). The eluate contains prekallikrein and kallikrein, but not kallikrein-Cl-inhibitor complex.

Aliquot B is incubated with an excess of Cl-inhibitor for ten minutes at 24°C and then passed through the column as for aliquot A. The eluate will contain only prekallikrein.

A monoclonal antibody according to the present invention, which recognizes an antigenic determinant shared by prekallikrein, kallikrein and kallikrein-Cl-inhibitor complex, is then separately contacted with the thus-treated aliquots A and B, and with the non-treated aliquot C. The material bound by the antibody comprises, respectively, prekallikrein plus kallikrein in aliquot A; prekallikrein in aliquot B; and prekallikrein plus kallikrein plus kallikrein-Cl-inhibitor complex in C. The bound material is measured by means of standard assay methods. Such methods include, but are not limited to, immunological assay methods such as radioimmunoassay, ELISA, fluorescent assay, precipitation, agglutination, and electroimmunodiffusion. Such methods also include fast liquid chromatographic methods such as ion exchange, gel filtration and reverse phase chromatography.

The instant monoclonal antibodies are thus useful to measure prekallikrein, kallikrein and kallikrein-Cl-inhibitor complex levels in the plasma in pathogenic states related to the contact activation of the coagulation pathway. These pathogenic states include, but are not limited to, acquired disorders such as gram negative or positive sepsis, carcinoid syndrome, postgastrectomy syndrome, nephrotic syndrome, type IIa hyperlipoproteinemia, allograft rejection, allergic reactions, typhoid fever, viremia and Rocky Mountain Spotted Fever. The monoclonal antibodies are also useful in measuring abnormal prekallikrein and kallikrein levels connected with hereditary disorders such as hereditary angioedema.

The monoclonal antibodies of the present invention are useful in removing prekallikrein and kallikrein from the blood, thereby providing a method of therapy for disease conditions wherein the patient suffers from increased levels of these contact activation proteins. Following removal of excessive amounts of kallikrein and prekallikrein, the blood may be returned to the body. Moreover, since the instant monoclonal antibodies have been demonstrated to inhibit the activity of prekallikrein and kallikrein in the contact activation of the coagulation pathway, these antibodies or their Fab fragments, may be used to reduce the level of functional kallikrein in vivo by infusion into the blood stream.

The monoclonal antibodies of the present invention may be easily covalently bound to an immobilized matrix such as CNBr-activated Sephrose, as disclosed in Methods In Enzymology 82:87 (1980). Prekallikrein may be purified by passing plasma through such a column containing immobilized monoclonal antibody, and then elluting the bound prekallikrein. Purification of prekallikrein in this manner may take place with or without prior partial purification, such as by ion exchange chromatography.

A column of the type described above may also be used to separate the light and heavy chains of kallikrein since the antibodies of the present invention are specific for an epitope of kallikrein heavy chain. Moreover, because prekallikrein binds high molecular weight kininogen, and because these plasma proteins do not compete for binding of the present monocolonal antibodies, columns of this type charged with prekallikrein may be used to purify high molecular weight kininogen. Finally, since the present antibodies recognize kallikrein even after complexing with Cl-inhibitor, they may be used to separate kallikrein-Cl-inhibitor complex from other components.

The preparation of the hybridomas of the present invention and the production, purification and characterization of the resulting monoclonal antibodies may be carried out as above. Although the subject monoclonal antibodies were prepared by intraperitoneal injection of hybridomas into mice and harvesting of blood or ascites, antibody may also be obtained by culturing the hybridomas by in vitro techniques known to those skilled in the art.

Three of the four reported hybridomas, namely 6A6, 13Hll, and 13Gll, belong to sub-class IgG₁, which means they have the same "constant" region. An antibody to a specific antigen has a "variable" region which functionally recognizes the antigen and a constant region. The variable region recognizes antigen regardless of the type of constant region. Thus, monoclonal antibodies exhibiting the characteristics described herein may be of sub-class IgG₁, IgG₂ₐ, IgG_{2b}, IgG₃, IgM, IgA or other Ig classes. Since the difference in immunoglobulin class (Ig) will not affect the pattern of reactivity of the antibody toward the antigen, it is contemplated that monoclonal antibodies to human plasma prekallikrein are included within the subject invention regardless of Ig class of sub-class.

The method of the present invention for preparing monoclonal antibodies to human plasma prekallikrein, which include immunization, fusion and selection of hybridomas, may be followed to generate cell lines other than the four cell lines disclosed herein. Because individual hybridomas may be identified only by the antibody which they produce, it is contemplated that any hybridoma-producing antibody to human plasma prekallikrein is included within the scope of the present invention, as are methods for making such antibodies employing hybridomas. It is further contemplated that splenocytes and myelomas from other vertebrates, such as human, rat, bovine, porcine, etc., not just those of murine origin, may be used to form hybridomas using the methods described herein.

The monoclonal antibodies of the present invention are produced by hybridomas. However, it is contemplated that other methods of cell-immortalization may be used to produce monoclonal antibodies against human plasma prekallikrein. These methods are known to those skilled in the art. For example, human antibody-producing lymphocytes may be immortalized by transformation with Epstein-Barr virus. See, e.g., Chiorazzi et al., J. Exp. Med. 56, 930-35 (1982), Steintz, et al., J. Immunol. 132, 877-82 (1984).

## Claims

1. A monoclonal antibody which specifically binds to an antigenic determinant of human plasma prekallikrein and inhibits the procoagulant activity of such prekallikrein.

2. A monoclonal antibody which binds to a single antigenic determinant shared by prekallikrein, kallikrein and kallikrein-C1-inhibitor complex.

3. A monoclonal antibody according to claim 1 or 2 which binds to plasma kallikrein but not tissue kallikrein.

4. A monoclonal antibody according to claim 1 or 2 which inhibits the enzyme activity of kallikrein.

5. A monoclonal antibody according to any preceding claim which is specific for the heavy chain of kallikrein.

6. A monoclonal antibody according to any preceding claim produced by a hybridoma formed by fusion of cells from a myeloma line and spleen cells from a donor previously immunized with human plasma prekallikrein.

7. A monoclonal antibody according to claim 6 wherein the myeloma line and spleen cells are murine.

8. A monoclonal according to claim 7 wherein the hybridoma is formed by fusion of SP2/0-Agl4 myeloma cells and spleen cells from BALB/c AnSkh mouse previously immunized with purified human plasma prekallikrein.

9. A non-therapeutic method for producing a monoclonal antibody which binds to an antigenic determinant of human plasma prekallikrein comprising the steps of:
(a) immunizing a splenocyte donor with purified human plasma prekallikrein;
(b) removing the spleens from said donor and making a suspension of the spleen cells;
(c) fusing said spleen cells with myeloma cells;
(d) diluting and culturing the fused cells in separate wells in a medium which will not support the unfused myeloma cells;
(e) assaying the supernatant in each well containing a hybridoma for the presence of antibody to human plasma prekallikrein; and
(f) selecting and cloning a hybridoma-secreting antibody which binds to human plasma prekallikrein, the procoagulant activity of such that prekallikrein is inhibited and/or the antigenic determinant so bound is shared by prekallikrein, kallikrein and kallikrein-Cl-inhibitor complex.

10. A method according to claim 9 wherein the donor cells and the myeloma cells are murine.

11. A method according to claim 10 comprising the further steps of transferring said clones into mice and harvesting the malignant ascites or serum from said mice, said ascites or serum containing the desired antibody.

12. A method according to claim 10 comprising the further steps of culturing the hybridoma in a suitable medium and recovering the antibody from the culture supernatant.

13. A method for producing a monoclonal antibody against human plasma prekallikrein which comprises culturing a hybridoma selected from the group consisting of four hybridomas numbered consecutively from ATCC #HB-8862 to ATCC #HB-8865, and recovering the secreted monoclonal antibodies from the culture medium.

14. A monoclonal antibody obtainable by the method of claim 13.

15. A non-therapeutic method for producing a monoclonal antibody against human plasma prekallikrein which comprises injecting into a mouse a hybridoma selected from the group consisting of four hybridomas numbered consecutively from ATCC #HB-8862 to ATCC #HB-8865, and recovering the secreted monoclonal antibodies from the mouse ascitic fluid or serum.

16. A monoclonal antibody obtainable by the method of claim 15.

17. A composition comprising a continuous cell line producing monoclonal antibodies according to claim 1 or 2 comprising a cell hybrid of a spleen cell from a donor previously immunized with human plasma prekallikrein fused to a myeloma, and a culture medium for said hybrid.

18. The composition according to claim 17 wherein the spleen cell and myeloma are murine.

19. The composition according to claim 18 wherein the spleen cell is from a BALB/c AnSkh mouse.

20. The composition according to claim 18 wherein the myeloma is SP2/0-Agl4.

21. A cell line selected from the group of cell lines numbered consecutively from ATCC #HB-8862 to ATCC #HB-8865.

22. A method for determining the level of human plasma prekallikrein, kallikrein and kallikrein-Cl-inhibitor complex in a specimen comprising:
(a) dividing said specimen into two aliquots, A and B;
(b) incubating B with excess Cl-inhibitor to convert all kallikrein in B to kallikrein-Cl-inhibitor complex;
(c) separating the proteins of A according to molecular weight;
(d) separating the proteins of B according to molecular weight;
(e) contacting said separated proteins of A and B with a monoclonal antibody which binds to a single antigenic determinant shared by prekallikrein, kallikrein and kallikrein-Cl-inhibitor complex; and
(f) measuring the protein bound by said antibody by an assay means.

23. A method according to claim 22 wherein step (f) comprises:
measuring the amount of about 185 kDa protein from A bound by said antibody to indicate the amount of kallikrein-Cl-inhibitor complex in said specimen;
measuring the amount of about 85-88 kDa protein from B bound by said antibody to indicate the amount of prekallikrein in said specimen; and
measuring the amount of about 85-88 kDa protein from A bound by said antibody, and subtracting therefrom the amount of about 85-88 kDa protein from B bound by said antibody to indicate the amount of kallikrein in said specimen.

24. A method according to claim 22 or 23 wherein the specimen is plasma.

25. A method according to claim 22, 23 or 24 wherein separation of proteins in steps (c) and (d) is by electrophoresis.

26. A method according to any of claims 22 to 25 wherein the assay means is selected from the group consisting of radioimmunoassay, enzyme-linked immunosorbent assay, and combinations thereof.

27. A method according to any of claims 22 to 25 wherein the monoclonal antibody is produced by a hybridoma selected from the group of the hybridomas numbered consecutively from ATCC #HB-8862 to ATCC #HB-8864.

28. A monoclonal antibody against human plasma prekallikrein obtainable from the hybridoma cell line having the accession number ATCC #HB-8862, ATCC #HB-8863, ATCC #HB-8864 or ATCC #HB-8865.

29. A monoclonal antibody which specifically binds to an antigenic determinant of human plasma prekallikrein, which antigenic determinant is characterized by being recognized by a monoclonal antibody produced by any of hybridomas ATCC #HB-8862, -8863, -8864 or -8865.

30. A composition comprising a continuous cell line producing monoclonal antibody which specifically binds to an antigenic determinant of human plasma prekallikrein which antigenic determinant is characterized by being recognized by a monoclonal antibody produced by any of hybridomas ATCC #HB-8862, -8863, -8864 or -8865.

## Patentansprüche

1. Monoklonaler Antikörper, welcher spezifisch an eine antigene Determinante von Humanplasma-Präkallikrein bindet und die die Blutgerinnung begünstigende Aktivität eines derartigen Präkallikreins hemmt.

2. Monoklonaler Antikörper, welcher an eine einzelne antigene Determinante bindet, welche von Präkallikrein, Kallikrein und dem Kallikrein-Cl-Hemmer-Komplex geteilt wird.

3. Monoklonaler Antikörper gemäß Anspruch 1 oder 2, welcher an Plasma-Kallikrein bindet, nicht aber an Gewebe-Kallikrein.

4. Monoklonaler Antikörper gemäß Anspruch 1 oder 2, welcher die Enzymaktivität von Kallikrein hemmt.

5. Monoklonaler Antikörper gemäß irgendeinem vorangehenden Anspruch, welcher für die schwere Kette von Kallikrein spezifisch ist.

6. Monoklonaler Antikörper gemäß irgendeinem vorangehenden Anspruch, der durch ein Hybridom produziert wird, das durch Zellfusion einer Myelomlinie und Milzzellen aus einem Spender gebildet wird, welcher mit Humanplasma-Präkallikrein immunisiert wurde.

7. Monoklonaler Antikörper gemäß Anspruch 6, bei welchem die Myelomlinie und die Milzzellen von der Maus sind.

8. Monoklonaler Antikörper gemäß Anspruch 7, bei welchem das Hybridom durch Fusion von SP2/0-Agl4-Myelomzellen und Milzzellen aus einer BALB/c AnSkh-Maus gebildet ist, welche zuvor mit gereinigtem Humanplasma-Präkallikrein immunisiert wurde.

9. Nicht-therapeutisches Verfahren zum Herstellen eines monoklonalen Antikörpers, welcher an eine antigene Determinante von Humanplasma-Präkallikrein bindet, umfassend die Schritte des
(a) Immunisierens eines Splenozyten-Spenders mit gereinigtem Humanplazma-Präkallikrein,
(b) Entnehmen der Milzen aus dem Spender und Bereiten einer Suspension aus den Milzzellen,
(c) Fusionieren der Milzzellen mit Myelomzellen,
(d) Verdünnen und Kultivieren der fusionierten Zellen in getrennten Näpfchen in einem Medium, das die nicht-fusionierten Myelomzellen nicht unterstützt,
(e) Testen des Überstands in jedem ein Hybridom enthaltenden Näpfchen auf die Anwesenheit eines Antikörpers gegenüber Humanplazma-Präkallikrein und
(f) Selektieren und Klonieren eines Hybridom-sekretierenden Antikörpers, welcher an Humanplasma-Präkallikrein bindet, dessen die Blutgerinnung begünstigende Aktivität derart ist, daß Präkallikrein gehemmt wird und/oder die auf diese Weise gebundene antigene Determinante von Präkallikrein, Kallikrein und dem Kallikrein-Cl-Hemmer-Komplex geteilt wird.

10. Verfahren gemäß Anspruch 9, bei welchem die Spenderzellen und die Myelomzellen von der Maus sind.

11. Verfahren gemäß Anspruch 10, welches die weiteren Schritte des Überführenz der Klone in Mäuse und des Erntens des malignen Aszites oder des Serums aus den Mäusen umfaßt, wobei der Aszites oder das Serum den gewünschten Antikörper enthält.

12. Verfahren gemäß Anspruch 10, welches die weiteren Schritte des Kultivierens des Hybridoms in einem geeigneten Medium und des Gewinnens des Antikörpers aus dem Kulturüberstand umfaßt.

13. Verfahren zum Herstellen eines monoklonalen Antikörpers gegen Humanplasma-Präkallikrein, welches das Kultivieren eines Hybridoms, das aus der Gruppe ausgewählt ist, welche aus vier fortlaufend von ATCC #HB-8862 bis ATCC #HB-8865 numerierten Hybridomen besteht, und das Gewinnen der sekretierten monoklonalen Antikörper aus dem Kulturmedium umfaßt.

14. Monoklonaler Antikörper, der durch das Verfahren von Anspruch 13 erhältlich ist.

15. Nicht-therapeutisches Verfahren zum Herstellen eines monoklonalen Antikörpers gegen Humanplasma-Präkallikrein, welches das Injizieren eines Hybridoms, das aus der Gruppe ausgewählt ist, welche aus vier fortlaufend von ATCC #HB-8862 bis ATCC #HB-8865 numerierten Hybridomen besteht, in eine Maus und das Gewinnen der sekretierten monoklonalen Antikörper aus dem Kulturmedium umfaßt.

16. Monoklonaler Antikörper, der durch das Verfahren von Anspruch 15 erhältlich ist.

17. Zusammensetzung umfassend eine kontinuierliche Zellinie, welche monoklonale Antikörper gemäß Anspruch 1 oder 2 produziert, die ein Zellhybrid einer Milzzelle aus einem Spender umfaßt, welcher zuvor mit Humanplasma-Präkallikrein immunisiert wurde, das mit einem Myelom fusioniert wurde, und ein Kulturmedium für das Hybrid.

18. Zusammensetzung gemäß Anspruch 17, in welcher die Milzzelle und das Myelom von der Maus sind.

19. Zusammensetzung gemäß Anspruch 18, in welcher die Milzzelle von einer BALB/c AnSkh-Maus ist.

20. Zusammensetzung gemäß Anspruch 18, in welcher das Myelom SP2/0-Agl4 ist.

21. Zellinie, die aus der Gruppe von Zellinien ausgewählt ist, welche fortlaufend von ATCC #HB-8862 bis ATCC #HB-8865 numeriert sind.

22. Verfahren zum Bestimmen des Gehalts von Humanplasma-Präkallikrein, Kallikrein und dem Kallikrein-Cl-Hemmer-Komplex in einer Probe, umfassend
(a) Teilen der Probe in zwei aliquote Anteile A und B,
(b) Inkubieren von B mit überzchüssigem Cl-Hemmer zum Überführen allen Kallikreins in B in den Kallikrein-Cl-Hemmer-Komplex,
(c) Trennen der Proteine von A entsprechend dem Molekulargewicht,
(d) Trennen der Proteine von B entsprechend dem Molekulargewicht,
(e) Zusammenbringen der abgetrennten Proteine von A und B mit einem monoklonalen Antikörper, der an eine einzige antigene Determinante bindet, welche von Präkallikrein, Kallikrein und dem Kallikrein-Cl-Hemmer-Komplex geteilt wird, und
(f) Messen des durch den Antikörper gebundenen Proteins durch ein Testmittel.

23. Verfahren gemäß Anspruch 22, bei welchem Schritt (f) das
Messen der Menge des von dem Antikörper gebundenen Proteins aus A mit etwa 185 kDa, um die Menge Kallikrein-Cl-Hemmer-Komplex in der Probe anzuzeigen,
Messen der Menge des von dem Antikörper gebundenen Proteins aus B mit etwa 85-88 kDa, um die Menge Präkallikrein in der Probe anzuzeigen, und
das Messen der Menge des von dem Antikörper gebundenen Proteins aus A mit etwa 85-88 kDa, und das Abziehen der Menge des von dem Antikörper gebundenen Proteins aus B mit etwa 85-88 kDa davon, um die Menge Kallikrein in der Probe anzuzeigen.

24. Verfahren gemäß Anspruch 22 oder 23, bei welchem die Probe Plasma ist.

25. Verfahren gemäß Anspruch 22, 23 oder 24, bei welchem die Trennung der Proteine in Schritt (c) und (d) durch Elektrophorese erfolgt.

26. Verfahren gemäß einem der Ansprüche 22 bis 25, bei welchem das Testmittel aus der Gruppe ausgewählt wird, welche aus einer Radioimmunbestimmung, einer enzymverbundenen Immunadsorptionsbestimmung und deren Kombinationen besteht.

27. Verfahren gemäß einem der Ansprüche 22 bis 25, bei welchem der monoklonale Antikörper durch ein Hybridom produziert wird, das aus der Gruppe von Hybridomen ausgewählt ist, welche fortlaufend von ATCC #HB-8862 bis ATCC #HB-8865 numeriert sind.

28. Monoklonaler Antikörper gegen Humanplasma-Präkallikrein, der aus der Hybridom-Zellinie mit der Zugangsnummer ATCC #HB-8862, ATCC #HB-8863, ATCC #HB-8864 oder ATCC #HB-8865 erhältlich ist.

29. Monoklonaler Antikörper, welcher spezifisch an eine antigene Determinante von Humanplasma-Präkallikrein bindet, welche dadurch gekennzeichnet ist, daß sie von einem monoklonalen Antikörper erkannt wird, der von einem der Hybridome ATCC #HB-8862, -8863, -8864 oder -8865 produziert wird.

30. Zusammensetzung umfassend eine kontinuierliche Zellinie, die einen monoklonalen Antikörper produziert, der spezifisch an eine antigene Determinante von Humanplasma-Präkallikrein bindet, welche dadurch gekennzeichnet ist, daß sie von einem monoklonalen Antikörper erkannt wird, der von einem der Hybridome ATCC #HB-8862, -8863, -8864 oder -8865 produziert wird.

## Revendications

1. Anticorps monoclonal oui se lie spécifiquement à un déterminant antigénique de prékallicréine de plasma humain et oui inhibe l'activité procoagulante d'une telle prékallicréine.

2. Anticorps monoclonal qui se lie à un déterminant antigénique unique porté par la prékallicréine, la kallicréine et le complexe kallicréine/inhibiteur Cl.

3. Anticorps monoclonal selon la revendication 1 ou 2, qui se lie à la kallicréine plasmatique mais pas à la kallicréine tissulaire.

4. Anticorps monoclonal selon la revendication 1 ou 2, qui inhibe l'activité enzymatique de la kallicréine.

5. Anticorps monoclonal selon l'une des revendications précédentes qui est spécifique pour la chaîne lourde de la kallicréine.

6. Anticorps monoclonal selon l'une des revendications précédentes produite par un hybridome formé par fusion de cellules à partir d'une lignée de myélome et de cellules de rate provenant d'un donneur préalablement immunisé avec de la prékallicréine de plasma humain.

7. Anticorps monoclonal selon la revendication 6, caractérisé en ce que la lignée de myélome et les cellules de rate sont d'origine murine.

8. Anticorps monoclonal selon la revendication 7, caractérisé en ce que l'hybridome est formé par fusion de cellules de myélome SP 2/0-Agl4 et de cellules de rate provenant de souris BALB/C AnSkh préalablement immunisées avec de la prékallicréine de plasma humain purifiée.

9. Méthode non thérapeutique de production d'un anticorps monoclonal qui se lie à un déterminant antigénique de prékallicréine de plasma humain qui comprend les étapes de :
a) immunisation d'un donneur de splenocytes avec de la prékallicréine de plasma humain purifiée,
b) éliminant les rates du dit donneur et en faisant une suspension des cellules spléniques,
c) fusionnant les dites cellules spléniques avec les cellules de myélome,
d) dilution et mise en culture des cellules fusionnées dans des puits séparés avec un milieu qui ne favorise pas les cellules de myélome non fusionnées,
e) l'essai du surnageant dans chaque puits contenant un hybridome, pour détecter la présence d'un anticorps contre la prékallicréine de plasma humain, et
f) le choix et le clonage d'un anticorps sécrétant un hybridome qui se lie à la prékallicréine de plasma humain, dont l'activité procoagulante d'une telle prékallicréine est inhibée et/ou le déterminant antigénique ainsi lié est porté par la prékallicréine, la kallicréine et le complexe kallicréine/inhibiteur Cl.

10. Méthode selon la revendication 9, caractérisée en ce que les cellules de donneur et les cellules de myélome sont d'origine murine.

11. Méthode selon la revendication 10 qui comprend les étapes ultérieures de transfert des dits clones dans la souris et de recueil d'ascite ou le sérum maligne à partir des dites souris, les dites ascites ou sérum, contenant l'anticorps désiré.

12. Méthode selon la revendication 10, qui comprend les étapes ultérieures de mise en culture de l'hybridome dans un milieu approprié et de la récupération de l'anticorps du surnageant de la culture.

13. Méthode de production d'un anticorps monoclonal contre la prékallicréine de plasma humain qui comprend la mise en culture d'un hybridome choisi dans le groupe formé des quatre hybridomes numérotés consécutivement de ATCC #HB-8862 à ATCC #HB-8865 et la récupération des anticorps monoclonaux sécrétés, à partir du milieu de culture.

14. Anticorps monoclonal que l'on peut obtenir par la méthode de la revendication 13.

15. Méthode non thérapeutique de production d'un anticorps monoclonal contre la prékallicréine de plasma humain qui comprend l'injection à des souris d'un hybridome choisi dans le groupe formé des quatre hybridomes numérotés consécutivement de ATCC #HB-8862 à ATCC #HB-8865, et la récupération des anticorps monoclonaux sécrétés à partir du fluide d'ascite ou du sérum de souris.

16. Anticorps monoclonal que l'on peut obtenir par la méthode de la revendication 15.

17. Composition qui comprend une lignée cellulaire continue qui produit des anticorps monoclonaux selon la revendication 1 ou la revendication 2, comprenant une cellule hybride d'une cellule splénique provenant d'un donneur préalablement immunisé avec de la prékallicréine de plasma humain, fusionnée à un myélome et un milieu de culture pour cet hybride.

18. Composition selon la revendication 17, caractérisée en ce que la cellule splénique et le myélome sont d'origine murine.

19. Composition selon la revendication 18, caractérisée en ce que les cellules spléniques proviennent de souris BALB/C AnSkh.

20. Composition selon la revendication 18, caractérisée en ce que le myélome est SP2/0-Agl4.

21. Lignée cellulaire choisie dans le groupe de lignée cellulaires numérotées consécutivement de ATCC #HB-8862 à ATCC #HB-8865.

22. Méthode de détermination du taux de prékallicréine, de kallicréine et de complexe kallicréine/inhibiteur Cl dans un échantillon qui comprend :
a) division du dit échantillon en deux aliquotes A et B,
b) incubation de B avec un excès d'inhibiteur Cl pour convertir toute la kallicréine dans B en complexe kallicréine/inhibiteur Cl,
c) séparation des protéines de A en fonction du poids moléculaire,
d) séparation des protéines de B en fonction du poids moléculaire,
e) mise en contact des dites protéines séparées de A et de B avec un anticorps monoclonal qui se lie à un déterminant antigénique unique porté par la prékallicréine/inhibiteur Cl, et
f) mesure de la protéine liée par ledit anticorps par des moyens d'essai.

23. Méthode selon la revendication 22, caractérisée en ce que l'étape f) comprend :
- la mesure de la quantité de protéine d'environ 185 kDa liée par ledit anticorps pour indiquer la quantité de complexe kallicréine/inhibiteur Cl dans le dit échantillon,
- la mesure de la quantité de protéine d'environ 85-88 kDa provenant de B liée au dit anticorps pour indiquer la quantité de prékallicréine dans le dit échantillon et,
- la mesure de la quantité de protéine d'environ 85-88 kDa provenant de A liée par ledit anticorps pour indiquer la quantité de kallicréine dans le dit échantillon.

24. Méthode selon la revendication 22 ou 23, dans laquelle l'échantillon est du plasma.

25. Méthode selon les revendications 22, 23 ou 24, caractérisée en ce que la séparation des protéines aux étapes c) et d) s'effectue par électrophorèse.

26. Méthode selon l'une des revendications 22 à 25, caractérisée en ce que les moyens d'essai sont choisis dans le groupe formé du radio-immuno-essai, l'essai d'immunosorption liée à une enzyme et les combinaisons de ceux-ci.

27. Méthode selon l'une des revendications 22 à 25, caractérisée en ce que l'anticorps monoclonal est produit par un hybridome choisi dans le groupe formé des hybridomes numérotés consécutivement de ATCC #HB-8862 à ATCC #HB-8864.

28. Anticorps monoclonal contre la prékallicréine de plasma humain que l'on peut obtenir de la lignée cellulaire d'hybridomes ayant le numéro d'accession ATCC #-8862, ATCC #-8863, ATCC #HB-8864 ou ATCC #HB-8865.

29. Anticorps monoclonal qui se lie spécifiquement à un déterminant antigénique de prékallicréine de plasma humain, ce déterminant antigénique étant caractérisé en ce qu'il est reconnu par un anticorps monoclonal produit par l'un des hybridomes ATCC #HB-8862, 8863, 8864 ou 8865.

30. Composition qui comprend une lignée cellulaire continue qui produit un anticorps monoclonal se liant spécifiquement à un déterminant antigénique de prékallicréine de plasma humain, ce déterminant antigénique est caractérisé en ce qu'il est reconnu par un anticorps monoclonal produit par l'un des hybridomes ATCC #HB-8862, 8863, 8864, ou 8865.
